# EUROPÄISCHE PATENTSCHRIFT

(11) **EP 3 606 397 B1**
(45) Veröffentlichungstag und Bekanntmachung des Hinweises auf die Patenterteilung: **17.03.2021**
(21) Anmeldenummer: 18717244.0
(22) Anmeldetag: 22.03.2018
(51) Int. Cl.: A61B 1/00, G02B 23/24, G02B 7/08, G02B 7/06, H02K 33/18, H01F 7/16

(54) **STEREOSKOPISCHES OPTISCHES SYSTEM EINES CHIRURGISCHEN INSTRUMENTS UND VERFAHREN ZUM HERSTELLEN DESSELBEN**
STEREOSCOPIC OPTICAL SYSTEM OF A SURGICAL INSTRUMENT AND METHOD FOR PRODUCING SAME
SYSTÈME OPTIQUE STÉRÉOSCOPIQUE D'UN INSTRUMENT CHIRURGICAL ET PROCÉDÉ POUR SA FABRICATION

(30) Priorität: 06.04.2017 DE 102017107414
(43) Veröffentlichungstag der Anmeldung: 12.02.2020
(73) Patentinhaber: Olympus Winter & Ibe GmbH, 22045 Hamburg (DE)
(72) Erfinder: WIETERS, Martin, 22885 Barsbüttel (DE)
(74) Vertreter: Seemann & Partner Patentanwälte mbB
(86) Internationale Anmeldenummer: PCT/EP2018/057351
(87) Internationale Veröffentlichungsnummer: WO 2018/184862

(56) Entgegenhaltungen:
- EP-A2- 2 947 756
- WO-A1-2016/012248

## Beschreibung

Die Erfindung betrifft ein stereoskopisches optisches System eines chirurgischen Instruments, umfassend einen linken optischen Kanal, einen rechten optischen Kanal und einen elektromagnetischen Aktuator mit einem Stator und einem Läufer. Ferner betrifft die Erfindung ein chirurgisches Instrument sowie ein Verfahren zum Herstellen eines stereoskopischen optischen Systems eines chirurgischen Instruments, mit einem linken optischen Kanal, einem rechten optischen Kanal und einem elektromagnetischen Aktuator mit einem Stator und einem Läufer.

Elektromagnetische Aktuatoren haben vielfältige Anwendungen. Beispielsweise können mit ihnen Schalter betätigt oder Mikrooptiken eingestellt oder verstellt werden. Bei chirurgischen Instrumenten, beispielsweise Endoskopen, können diese kleinbauenden Aktuatoren dazu verwendet werden, um einen Fokus oder eine Vergrößerung eines optischen Systems zu verändern. Bei Endoskopen mit variabler Blickrichtung ist es außerdem möglich, mithilfe eines elektromagnetischen Aktuators eine Blickrichtung des optischen Systems einzustellen oder zu verändern. Die Veränderung der optischen Eigenschaften eines optischen Systems erfolgt, indem ein optisches Bauteil, beispielsweise eine Linse, ein Prisma oder eine Blende, durch den Aktuator verschoben wird, wobei sich das optische Bauteil im oder am Läufer des Aktuators befindet.

Es sind bistabile und monostabile elektromagnetische Aktuatoren bekannt. Bei einem bistabilen elektromagnetischen Aktuator ist ein Läufer vorhanden, der in einem Permanentmagnetfeld in einer von zwei extremen Positionen (Endpositionen) gehalten wird und durch Schaltung eines elektromagnetischen Felds aus einer dieser beiden stabilen Position in die jeweils andere stabile Position überführt werden kann. Bei einem monostabilen elektromagnetischen Aktuator wird der Läufer von einem Magnetfeld, welches von einem oder mehreren Permanentmagneten erzeugt wird, stabil in seiner Ruheposition gehalten. Durch Anlegen eines von einer Magnetspule erzeugten elektromagnetischen Felds wird der Läufer aus dieser stabilen Ruheposition heraus bewegt. Bistabile Systeme sind für einen zweistufigen Betrieb mit leistunglos gehaltenen Endpositionen besonders geeignet. Monostabile Systeme sind hingegen für eine kontinuierliche Verstellung gut geeignet.

Wie bereits erwähnt, können elektromagnetische Aktuatoren zur Einstellung oder Verstellung eines optischen Systems eines chirurgischen Instruments eingesetzt werden. Das optische System eines Stereo-Endoskops umfasst zwei Objektive, die idealerweise simultan zueinander eingestellt oder fokussiert werden. Zu diesem Zweck wäre es möglich, zwei separate elektromagnetische Aktuatoren einzusetzen, wie sie ansonsten zur Verstellung einer einzelnen Optik eingesetzt werden. Dies ist jedoch mit hohen Kosten verbunden.

Außerdem ist es wünschenswert, wenn die optischen Achsen der beiden Objektive, die für die stereoskopische Abbildung eingesetzt werden, einen möglichst großen Abstand, den sogenannten Stereoabstand, zueinander einhalten. Ein großer Stereoabstand ermöglicht einen guten 3-D Effekt. Gleichzeitig ist eine lichtstarke Optik wünschenswert, so dass nach Möglichkeit Objektive mit einem möglichst großen Durchmesser verwendet werden sollen. Je größer die Optikelemente werden, desto kleiner wird die freie Distanz zwischen den Objektiven. Soll der Stereoabstand konstant gehalten werden, so nimmt der Durchmesser eines die beiden Objektive umgebenden Rohres zu. Außerdem müssen die elektromagnetischen Aktuatoren untergebracht werden. Die genannten Forderungen stehen also mehr oder weniger in unmittelbarem Widerspruch zu dem im Rohr des Endoskops vorhandenen beschränkten Bauraum.

Aus EP 2 947 756 A2 ist ein positionsgeregelter elektrodynamischer Linearantrieb bekannt, der einen Stator und einen Läufer umfasst. Das magnetische Feld, das von einem äußeren Läuferpolschuh erzeugt wird, verläuft in Abhängigkeit von der Läuferposition ganz oder nur teilweise durch zwei an den Statorpolschuhen angeordnete Magnetfeldsensoren und erzeugt somit ein positionsabhängiges Signal, das zur Regelung der Position des Läufers verwendet werden kann.

WO 2016/012248 A1 zeigt ein Stereovideoendoskop mit mehreren optischen Baugruppen. In einem ersten Teilvolumen des Bauraums ist ein optisches System mit einem linken und einem rechten optischen Kanal vorhanden. In einem zweiten Teilvolumen des Bauraums ist eine weitere optische Baugruppe vorhanden, welche durch Rotation um eine Längsaxialrichtung eines Schafts in das erste Teilvolum einschwenkbar ist.

Es ist eine Aufgabe der Erfindung ein stereoskopisches optisches System, ein chirurgisches Instrument mit einem stereoskopischen optischen System sowie ein Verfahren zum Herstellen eines stereooptischen optischen Systems anzugeben, welches im Hinblick auf die im Stand der Technik vorhandenen Nachteile verbessert ist, also insbesondere eine kompakte Bauform aufweist.

Die Aufgabe wird gelöst durch ein stereoskopisches optisches System eines chirurgischen Instruments, umfassend einen linken optischen Kanal, einen rechten optischen Kanal und einen elektromagnetischen Aktuator mit einem Stator und einem Läufer, wobei das stereoskopische optische System dadurch fortgebildet ist, dass optische Komponenten des linken optischen Kanals in einem linken Führungsrohr und optische Komponenten des rechten optischen Kanals in einem separaten rechten Führungsrohr angeordnet sind, wobei der Stator außerhalb der Führungsrohre angeordnet ist und der Läufer einen linken Läufer, in dem zumindest eine optische Komponente des linken optischen Kanals aufgenommen ist, und einen rechten Läufer, in dem zumindest eine optische Komponente des rechten optischen Kanals aufgenommen ist, umfasst, und wobei der linke und der rechte Läufer in einer Längsaxialrichtung des linken und rechten Führungsrohrs in dem jeweiligen Führungsrohr verschiebbar gelagert sind, wobei die Läufer jeweils wenigstens teilweise ein paramagnetisches und/oder ferromagnetisches Material umfassen und durch Beaufschlagung mit einem elektromagnetischen Feld in der Längsaxialrichtung verschiebbar sind, wobei der Stator einen distalen Permanentmagneten und einen proximalen Permanentmagneten umfasst, die in Längsaxialrichtung gegensinnig gepolt sind, und wobei der Stator eine elektrische Spule zum Erzeugen des elektromagnetischen Felds umfasst.

Das linke und das rechte Führungsrohr sind insbesondere parallel zueinander ausgerichtet, was bedeutet, dass eine Längsaxialrichtung des linken Führungsrohrs parallel zu einer Längsaxialrichtung des rechten Führungsrohrs orientiert ist. Sofern nicht zwischen dem linken und dem rechten Führungsrohr unterschieden werden muss, wird im Folgenden auch allgemein auf eine Längsaxialrichtung Bezug genommen, die in gleicher Richtung wie die Längsaxialrichtungen des linken und rechten Führungsrohrs orientiert ist. Es ist jedoch ebenso vorgesehen, dass das linke und das rechte Führungsrohr einen Winkel von beispielsweise 2° einschließen. Ferner insbesondere ist dieser Winkel zwischen der Längsaxialrichtung des linken Führungsrohrs und der Längsaxialrichtung des rechten Führungsrohrs kleiner als 5°. Wird auf lediglich eine Längsaxialrichtung Bezug genommen und schließen das linke und das rechte Führungsrohr einen Winkel ein, so liegt diese Längsaxialrichtung in der Mitte zwischen der Längsaxialrichtung des linken Führungsrohrs und der Längsaxialrichtung des rechten Führungsrohrs.

Das stereoskopische optische System gemäß Aspekten der Erfindung ist besonders vorteilhaft, da der rechte Läufer und der linke Läufer mithilfe eines einzigen und gemeinsamen elektromagnetischen Aktuators betätigt werden. Eine solche Konstruktion ist technisch einfach und außerdem kostengünstig zu realisieren. Sie nimmt außerdem lediglich einen sehr geringen Bauraum in Anspruch.

Der Einsatz separater Führungsrohre für den linken optischen Kanal und den rechten optischen Kanal ist auch aus den folgenden Gründen vorteilhaft. Es wäre denkbar, einen gemeinsamen Halter für beide optische Kanäle bzw. deren optische Komponenten vorzusehen. Ein solcher Halter müsste jedoch bezüglich Rotation um seine Längsaxialrichtung gesichert bzw. fixiert werden. Nur so könnte eine nachträgliche Verdrehung der Optik gegenüber den übrigen optischen Komponenten des stereoskopischen optischen Systems, beispielsweise der Bildsensoren, zuverlässig vermieden werden. Dies wäre beispielsweise durch Einsatz einer Passfeder möglich. Eine solche Lösung bringt jedoch wiederum neue technische Herausforderungen mit sich. Beispielsweise wäre eine Spielpassung erforderlich, um die gewünschte axiale Verschiebbarkeit zu ermöglichen. Auch die Verwendung eines Halters mit ovalem Querschnitt wäre möglich, der in einer passenden ovalen Bohrung eines Gleitrohres abgleitet. Der Nachteil einer solchen Konstruktion wäre jedoch, dass Bauteile mit ovalem Querschnitt stets aufwendig und weniger präzise zu fertigen sind.

Vor diesem Hintergrund hat es sich als vorteilhaft herausgestellt, je ein separates Führungsrohr für den linken Läufer und den rechten Läufer vorzusehen. Insbesondere sind die Führungsrohre im Querschnitt kreisrund.

Gemäß einer vorteilhaften Ausführungsform ist das stereoskopische optische System dadurch fortgebildet, dass ein distales Ende des Stators von einem distalen Statorpolschuh und ein in Längsaxialrichtung gegenüberliegendes proximales Ende von einem proximalen Statorpolschuh gebildet sind. Ferner ist insbesondere vorgesehen, dass der Stator außerdem einen zentralen Statorpolschuh umfasst, der in Längsaxialrichtung zwischen den Permanentmagneten angeordnet ist. Vorteilhaft ist ferner vorgesehen, dass der zentrale Statorpolschuh aus einem proximalen zentralen Stator-Teil-Polschuh und aus einem distalen zentralen Stator-Teil-Polschuh gebildet ist. Insbesondere ist zwischen dem distalen zentralen Stator-Teil-Polschuh und dem proximalen zentralen Stator-Teil-Polschuh ein Luftspalt vorhanden.

Ferner ist insbesondere vorgesehen, dass die Spule eine distale Spule und eine proximale Spule umfasst, wobei der distale Statorpolschuh, eine distale Spule, der distale Permanentmagnet und der distale zentrale Stator-Teil-Polschuh eine vorgefertigte distale Baugruppe bilden und der proximale zentrale Stator-Teil-Polschuh, eine proximale Spule, der proximale Permanentmagnet und der proximale Statorpolschuh eine vorgefertigte proximale Baugruppe bilden, wobei insbesondere die Bauteile der distalen und/oder der proximalen Baugruppe miteinander verklebt sind.

Die Verwendung von Statorpolschuhen erlaubt die Erhöhung des Wirkungsgrads des elektromagnetischen Aktuators durch eine verbesserte magnetische Flussführung. In der Folge können vorteilhaft größere Haltekräfte bereitgestellt oder geringere Stellströme eingesetzt werden. Der zentrale Statorpolschuh ist insbesondere dicker als die äußeren Statorpolschuhe, d. h. der distale Statorpolschuh oder der proximale Statorpolschuh. Beispielsweise weist der zentrale Statorpolschuh eine Materialstärke, gemessen in Längsaxialrichtung, auf, welche 1,2-fach bis doppelt so groß ist wie die in gleicher Richtung gemessene Materialstärke der äußeren Polschuhe.

Der Luftspalt zwischen dem distalen zentralen Stator-Teil-Polschuh und dem proximalen zentralen Stator-Teil-Polschuh erlaubt es, eine distale und eine proximale Baugruppe zu bilden, die nicht mechanisch miteinander verbunden sind. Da sich die Permanentmagneten der beiden Baugruppen aufgrund ihrer gegensinnigen Orientierung gegenseitig abstoßen, richten sich die beiden Baugruppen selbstständig und unabhängig von gegebenenfalls vorhandenen Bauteiltoleranzen an einem distalen und einem proximalen Anschlag aus. Vorteilhaft kann auf die Verwendung eines Klebstoffs zur Verbindung der beiden Baugruppen verzichtet werden. Soll dennoch eine mechanische Verbindung der beiden Baugruppen hergestellt werden, so wird ein Klebstoff eingesetzt, der vorteilhaft eine besonders geringe Volumenschrumpfung während des Aushärtens zeigt. Geeignet ist beispielsweise ein Klebstoff, der während des Aushärtens weniger als 5 % Volumen einbüßt.

Es ist ferner insbesondere vorgesehen, dass die distale Baugruppe und die proximale Baugruppe identisch zueinander aufgebaut sind. Um die gegensätzliche magnetische Orientierung der Permanentmagnete in dem elektromagnetischen Aktuator zu realisieren, ist beim Zusammenbau vorgesehen, dass entweder die proximale Baugruppe oder die distale Baugruppe gegenüber der jeweils anderen Baugruppe um 180° gedreht eingebaut wird. Die Baugruppen werden passend verdrahtet, so dass diese gleich orientierte Magnetfelder erzeugen. Die Verwendung vorgefertigter Baugruppen beschleunigt die Herstellung des elektromagnetischen Aktuators.

Gemäß einer weiteren vorteilhaften Ausführungsform ist das stereoskopische optische System dadurch fortgebildet, dass das linke und das rechte Führungsrohr in einem gemeinsamen Bauteil aufgenommen sind, welches in einer Ebene quer zu der Längsaxialrichtung einen hantelförmigen Querschnitt aufweist und wobei eine Innenkontur der Polschuhe einer Außenkontur des hantelförmigen Bauteils entspricht und eine Außenkontur der Polschuhe zumindest abschnittsweise kreissegmentförmig ist.

Vorteilhaft werden die beiden optischen Kanäle, d. h. die beiden Führungsrohre, in einem gemeinsamen Bauteil aufgenommen oder durch ein solches Bauteil bereitgestellt. Um einen hohen Wirkungsgrad des Aktuators zu erreichen ist es notwendig, dass die Statorpolschuhe so nah wie möglich am Läufer liegen. Beispielsweise ist vorgesehen, dass die Statorpolschuhe ebenfalls eine Geometrie aufweisen, in welche zwei Bohrungen zur Aufnahme der jeweiligen Aufnahmerohre eingebracht sind. Es ergibt sich bei einer solchen Konstruktion jedoch ein sehr schmaler Steg zwischen den beiden Bohrungen, mit den entsprechenden mechanischen Instabilitäten. Es hat sich aus diesem Grund als vorteilhaft herausgestellt, wenn das zentrale Bauteil, in welchem die beiden Aufnahmerohre aufgenommen sind, hantelförmig ausgeführt wird. Dabei reichen die Statorpolschuhe bis an die äußere Oberfläche dieses hantelförmigen Bauteils heran. So wird einerseits eine große mechanische Stabilität erreicht, was den Zusammenbau des Systems vereinfacht. Gleichzeitig sind die Statorpolschuhe ausreichend nah an den Läufern positioniert, so dass eine effiziente magnetische Flussführung gewährleistet werden kann.

Ferner ist insbesondere vorgesehen, dass sich die Statorpolschuhe in einer zu der Längsaxialrichtung senkrechten Radialrichtung von einer Außenseite des hantelförmigen Bauteils bis zu einer dem hantelförmigen Bauteil abgewandten Außenseite der Permanentmagneten und/oder bis zu einer dem hantelförmigen Bauteil abgewandten Außenseite der Magnetspulen erstrecken. Vorteilhaft wird durch eine solche Ausgestaltung die Flussführung verbessert.

Gemäß einer weiteren Ausführungsform ist vorgesehen, dass die Spule eine distale Spule und eine proximale Spule umfasst, wobei sich die beiden Spulen in Längsaxialrichtung zu beiden Seiten des zentralen Statorpolschuhs erstrecken und elektrisch so miteinander gekoppelt sind, dass die distale Spule ein erstes Magnetfeld erzeugt, welches gleich zu einem von der proximalen Spule erzeugten zweiten Magnetfeld orientiert ist. Durch die Aufteilung der Magnetspule in eine erste Spule und eine zweite Spule kann diese in die vorgefertigte proximale Baugruppe und die vorgefertigte distale Baugruppe integriert werden.

In einer weiteren vorteilhaften Ausführungsform ist das stereoskopische optische System dadurch fortgebildet, dass die Spule das linke und das rechte Führungsrohr umgibt und in einer quer zu der Längsaxialrichtung gelegenen Ebene oval ist. Die Spule hat beispielsweise die Form zweier Halbkreissegmente zwischen deren auf einer Seite liegenden Enden jeweils gerade Stücke eingefügt sind. Die Form der Spule entspricht also mit anderen Worten beispielsweise der Form einer Laufbahn. Eine solche Geometrie der Spule ist mit vertretbarem Aufwand zu wickeln und erlaubt trotzdem eine effiziente Einkopplung des magnetischen Flusses in die entscheidenden Bereiche des elektromagnetischen Aktuators. Die Form der Spule ist ferner insbesondere an eine Außenkontur der Führungsrohre angepasst. Es wäre möglich, einzelne Spulen für jeden optischen Kanal zu verwenden. Eine ovale Spule, die für beide Führungsrohre gemeinsam wirkt, stellt jedoch eine wesentlich kostengünstigere und außerdem platzsparende Lösung dar.

Gemäß einer weiteren vorteilhaften Ausführungsform ist vorgesehen, dass die Permanentmagneten auf einer den Führungsrohren abgewandten Außenseite der Spule angeordnet sind. Eine solche Bauform ist besonders kompakt, da auf ein außenliegendes magnetisches Rückschlusselement verzichtet werden kann. Die Permanentmagneten wirken zumindest bereichsweise als magnetische Rückschlusselemente.

Ferner ist insbesondere vorgesehen, dass die Permanentmagneten blockförmige Magneten sind, die in zwei Gruppen angeordnet sind, wobei die Gruppen einander gegenüberliegend an je einer flachen Seite einer aus dem linken und rechten Führungsrohr gebildeten Anordnung angeordnet sind. An den flachen Seiten der neben einander liegenden Führungsrohren ist Bauraum vorhanden, da das Endoskoprohr, in dem die Einheit aufgenommen wird einen runden Innendurchmesser aufweist. Vorteilhaft ist es so möglich, die Optiken des rechten und linken Kanals möglichst weit voneinander entfernt zu positionieren, um so eine große Stereobasis zu realisieren. In dem verbleibenden Bauraum können die blockförmigen Permanentmagnete aufgenommen werden.

An den flachen Seiten der nebeneinander liegenden Rohre ist bei einer runden Aufnahmeöffnung freier Bauraum vorhanden. Diese flachen Seiten liegen zumindest näherungsweise parallel zu einem Abstand der beiden Rohre. An den senkrecht zu diesen flachen Seiten liegenden Stirnseiten, also in einer Ebene zumindest näherungsweise senkrecht zum Abstand zwischen den beiden Rohren, ist hingegen kein freier Bauraum vorhanden, da die beiden Rohre nebeneinander mit möglichst großem Abstand in der runden Aufnahmeöffnung platziert werden. Da die Spulen nur mit gleichmäßiger Wandstärke gewickelt werden können, ist es nicht möglich an diesen Stirnseiten der Anordnung Material der Spulen einzusparen. An den Stirnseiten kann jedoch auf Material des Stators und Material der Permanentmagnete verzichtet werden. Daher bietet sich die Nutzung der flachen Seiten zur Unterbringung der Permanentmagnete an. Es wäre möglich, Magnetscheiben zu verwenden, welche jedoch zumindest abschnittsweise eine sehr dünne Wandstärke aufweisen müssten. Da magnetisches Material ohnehin recht spröde ist, wäre die Handhabung derartiger Magnetscheiben beim Zusammenbau des stereoskopischen optischen Systems sehr schwierig. Magnetblöcke sind hingegen stabil und außerdem einfach und kostengünstig herzustellen. Ferner bewirkt die Anordnung der Magnete in dem genannten Bereich eine Kompensation der durch die Form der Statorpolschuhe beeinflussten magnetischen Flussführung.

Gemäß einer weiteren Ausführungsform ist vorgesehen, dass die Permanentmagneten magnetische Rückschlusselemente für das von der elektrischen Spule erzeugte Magnetfeld bilden. So kann vorteilhaft auf separate magnetische Rückschlusselemente verzichtet werden. Dies verringert die Bauform des elektromagnetischen Aktuators.

Gemäß einer weiteren vorteilhaften Ausführungsform ist das stereoskopische optische System dadurch fortgebildet, dass zumindest einer der Permanentmagnete hartmagnetische Partikel umfasst, die in einer Kunststoffmatrix eingebettet sind, wobei dieser Permanentmagnet insbesondere in einem Spritzgussverfahren hergestellt ist und ferner insbesondere in zumindest einem Permanentmagneten zumindest ein Spulendraht der Spule eingegossen ist. Als magnetische Partikel sind beispielsweise NdFeB-Partikel (Neodym, Eisen, Bohr) oder eine Mischung aus diesen Materialien geeignet, welche beispielsweise in einen Epoxidharzklebstoff eingerührt werden. Zur Herstellung der Permanentmagnete wird ein Freiraum zwischen den Statorpolschuhen ausgegossen. Dieser Freiraum wird anschließend von dem so hergestellten Permanentmagneten eingenommen. Vorteilhaft werden bei diesem Arbeitsgang nicht nur der Permanentmagnet selbst hergestellt sondern auch die Teile der Baugruppen miteinander verbunden bzw. vergossen. Anschließend ist beispielsweise vorgesehen, dass die so hergestellte Baugruppe aufmagnetisiert wird, so dass die magnetischen Partikel die gewünschte magnetische Orientierung einnehmen.

Die Aufgabe wird außerdem gelöst durch ein chirurgisches Instrument, insbesondere Endoskop, mit einem stereoskopischen optischen System nach einem oder mehreren der zuvor genannten Ausführungsformen.

Vorteilhaft kann das chirurgische Instrument ökonomisch und effizient hergestellt werden. Außerdem kann in einem solchen System eine große Stereobasis realisiert werden, was für die Abbildungseigenschaften eines chirurgischen Instruments, insbesondere eines Endoskops, besonders vorteilhaft ist. Im Übrigen treffen auf das chirurgische Instrument gleiche oder ähnliche Vorteile zu wie sie bereits im Hinblick auf das stereoskopische optische System selbst erwähnt wurden, so dass auf Wiederholungen verzichtet werden soll.

Die Aufgabe wird außerdem gelöst durch ein Verfahren zum Herstellen eines stereoskopischen optischen Systems eines chirurgischen Instruments, mit einem linken optischen Kanal, einem rechten optischen Kanal und einem elektromagnetischen Aktuator mit einem Stator und einem Läufer, dadurch gekennzeichnet, dass optische Komponenten des linken optischen Kanals in einem linken Führungsrohr und optische Komponenten des rechten optischen Kanals in einem separaten rechten Führungsrohr angeordnet werden, wobei der Stator außerhalb der Führungsrohre angeordnet wird und der Läufer einen linken Läufer, in dem zumindest eine optische Komponente des linken optischen Kanals aufgenommen wird, und einen rechten Läufer, in dem zumindest eine optische Komponente des rechten optischen Kanals aufgenommen wird, umfasst, und wobei der linke und der rechte Läufer in einer Längsaxialrichtung des linken und rechten Führungsrohrs in dem jeweiligen Führungsrohr verschiebbar gelagert werden, wobei die Läufer jeweils wenigstens teilweise ein paramagnetisches und/oder ferromagnetisches Material umfassen und durch Beaufschlagung mit einem elektromagnetischen Feld in der Längsaxialrichtung verschiebbar sind, wobei im Stator ein distaler Permanentmagnet und ein proximaler Permanentmagnet derart angeordnet werden, dass sie in Längsaxialrichtung gegensinnig gepolt sind, und wobei im Stator eine elektrische Spule zum Erzeugen des elektromagnetischen Felds angeordnet wird.

Auch auf das Verfahren zum Herstellen eines stereoskopischen optischen Systems treffen gleiche oder ähnliche Vorteile zu wie sie bereits im Hinblick auf das stereoskopische optische System selbst erwähnt wurden.

Gemäß einer vorteilhaften Ausführungsform ist das Verfahren dadurch fortgebildet, dass ein distales Ende des Stators von einem distalen Statorpolschuh und ein in Längsaxialrichtung gegenüberliegendes proximales Ende von einem proximalen Statorpolschuh gebildet sind und der Stator einen zentralen Statorpolschuh umfasst, der in Längsaxialrichtung zwischen den Permanentmagneten angeordnet wird und aus einem proximalen zentralen Stator-Teil-Polschuh und aus einem distalen zentralen Stator-Teil-Polschuh gebildet wird, wobei eine distale Baugruppe vorgefertigt wird, indem der distale Statorpolschuh, eine distale Spule, der distale Permanentmagnet und der distale zentrale Stator-Teil-Polschuh miteinander verklebt werden und eine proximale Baugruppe vorgefertigt wird, indem der proximale zentrale Stator-Teil-Polschuh, eine proximale Spule, der proximale Permanentmagnet und der proximale Statorpolschuh miteinander verklebt werden.

Dabei ist insbesondere vorgesehen, dass die Permanentmagneten blockförmige Magneten sind, wobei die Magneten in zwei Gruppen angeordnet werden und die Gruppe einander gegenüberliegend an je einer flachen Seite einer aus dem linken und rechten Führungsrohr gebildeten Anordnung angeordnet werden.

Ferner ist das Verfahren gemäß einer weiteren Ausführungsform dadurch fortgebildet, dass zumindest einer der Permanentmagneten hergestellt wird, indem hartmagnetische Partikel in eine Kunststoffmatrix eingebettet werden, wobei dieser Permanentmagnet insbesondere in einem Spritzgussverfahren hergestellt wird und ferner insbesondere in zumindest einem Permanentmagneten zumindest ein Spulendraht der Spule eingegossen wird.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein Endoskop als beispielhaftes chirurgisches Instrument in einer schematisch vereinfachten Perspektivansicht,
- Fig. 2: ein stereoskopisches optisches System in einer schematisch vereinfachten perspektivischen Ansicht,
- Fig. 3: dieses System, wobei ein proximaler Statorpolschuh entfernt ist, um den Blick auf die dahinterliegenden Bauteile freizugeben,
- Fig. 4: ein stereoskopisches optisches System in einer schematisch vereinfachten Schnittansicht in einer Ebene, in der eine Verbindungslinie der beiden optischen Kanäle liegt,
- Fig. 5: eine weitere schematische Schnittansicht in einer Ebene, welche senkrecht zu derjenigen Ebene liegt, in der die in Figur 4 dargestellte Schnittansicht liegt,
- Fig. 6 und 7: jeweils eine schematisch vereinfachte Prinzipskizze zur Erläuterung der Funktionsweise eines in einem stereoskopischen optischen System eingesetzten elektromagnetischen Aktuators.

In den Zeichnungen sind jeweils gleiche oder gleichartige Elemente und/oder Teile mit denselben Bezugsziffern versehen, so dass von einer erneuten Vorstellung jeweils abgesehen wird.

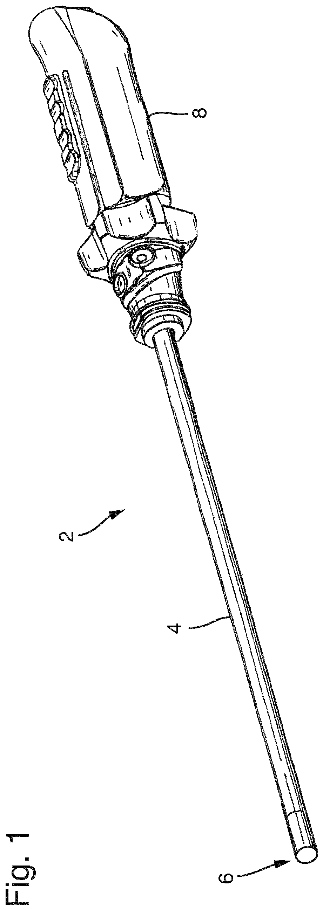

Figur 1 zeigt in einer schematisch vereinfachten Perspektivansicht ein Endoskop 2 als beispielhaftes chirurgisches Instrument. Das Endoskop 2 umfasst einen Endoskopschaft 4, in dem ein optisches System angeordnet ist, mit dem ein vor einem distalen Ende 6 des Endoskopschafts 4 liegendes Operations- oder Beobachtungsfeld abgebildet wird. An einem proximalen Ende des Endoskops 2 befindet sich ein Handgriff 8. Das in dem Endoskopschaft 4 angeordnete optische System, welches in Figur 1 nicht dargestellt ist, umfasst einen elektromagnetischen Aktuator.

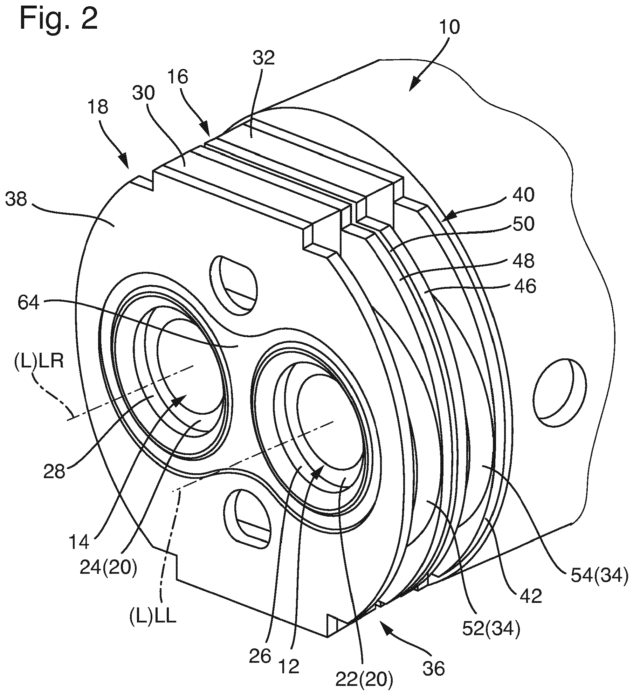

Figur 2 zeigt ein beispielhaftes optisches System 10, wie es im distalen Ende 6 des Endoskopschafts 4 des Endoskops 2 vorhanden sein kann. Bei dem optischen System 10 handelt es sich um ein stereoskopisches System, wie die in Figur 2 schematisch, vereinfacht und perspektivisch dargestellte Ansicht zeigt.

Figur 2 zeigt eine Einbausituation, bei der ein das stereoskopische optische System 10 im Normalfall umgebendes Endoskoprohr weggelassen ist, um den Blick auf die Bauteile des Systems freizugeben. Das stereoskopische optische System 10 umfasst einen linken optischen Kanal 12 und einen rechten optischen Kanal 14. Von den optischen Komponenten des linken optischen Kanals 12 und des rechten optischen Kanals 14 sind in Figur 2 beispielhaft die jeweiligen Frontlinsen der optischen Kanäle 12, 14 dargestellt. Das stereoskopische optische System 10 umfasst ferner einen elektromagnetischen Aktuator 16, umfassend einen Stator 18 und einen Läufer 20. Die optischen Komponenten des linken optischen Kanals 12, beispielsweise die in Figur 2 sichtbare Frontlinse, ist in einem linken Führungsrohr 26 aufgenommen. Dies betrifft zumindest einen Teil der optischen Komponenten des linken optischen Kanals 12. Optische Komponenten des rechten optischen Kanals 14 sind in einem zu dem linken Führungsrohr 26 separaten rechten Führungsrohr 28 angeordnet. Die beiden Führungsrohre 26, 28 sind beispielsweise parallel zueinander angeordnet. Es ist jedoch ebenso vorgesehen, dass die Führungsrohre 26, 28 in einem Winkel von beispielsweise 2° zueinander angeordnet sind, wobei dieser Winkel in der Regel 5° nicht übersteigt. Ebenso wie die optischen Komponenten des linken optischen Kanals sind auch die optischen Komponenten des rechten optischen Kanals 14 zumindest teilweise in dem rechten Führungsrohr 28 angeordnet. Bei den Führungsrohren 26, 28 handelt es sich beispielsweise um separate Bauteile, beispielsweise Rohre. Es ist ebenso vorgesehen, dass die Führungsrohre 26, 28 keine separaten Bauteile sind und stattdessen durch in ein hantelförmiges Bauteil 64 eingelassene Bohrungen bereitgestellt werden.

Der Stator 18 ist außerhalb der Führungsrohre 26, 28 angeordnet und umschließt die Führungsrohre 26, 28 vollständig. Dies gilt für eine zu einer Längsaxialrichtung der Führungsrohre 26, 28 senkrechte Richtung. In einer Längsaxialrichtung L ist es nicht erforderlich, dass der Stator 18 die Führungsrohre 26, 28 vollständig umschließt.

Der Läufer 20 umfasst einen linken Läufer 22, in dem zumindest eine optische Komponente des linken optischen Kanals 12 aufgenommen ist. Ferner umfasst der Läufer 20 einen rechten Läufer 24, in dem zumindest eine optische Komponente des rechten optischen Kanals 14 aufgenommen ist. Im dargestellten Ausführungsbeispiel sind jeweils die Frontlinsen der optischen Kanäle 12, 14 in den entsprechenden Läufern 22, 24 aufgenommen.

Der linke Läufer 22 ist in einer linken Längsaxialrichtung LL entlang des linken Führungsrohrs 26 verschiebbar gelagert. Der rechte Läufer 24 ist entlang einer rechten Längsaxialrichtung LR in dem rechten Führungsrohr 28 verschiebbar gelagert. Die linke Längsaxialrichtung LL und die rechte Längsaxialrichtung LR (jeweils mit strichpunktierter Linie angedeutet) sind parallel zueinander ausgerichtet. Sie fallen mit den Mittenlängsachsen der jeweiligen Führungsrohre 26, 28 zusammen. Sofern es im Folgenden nicht erforderlich ist, zwischen der linken Längsaxialrichtung LL und der rechten Längsaxialrichtung LR zu unterscheiden, wird allgemein auf eine Längsaxialrichtung L Bezug genommen, welche sich parallel zu der linken und der rechten Längsaxialrichtung LL, LR erstreckt.

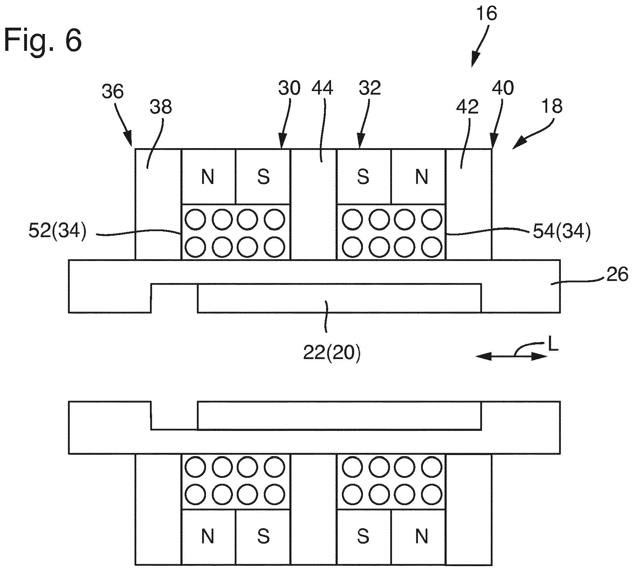

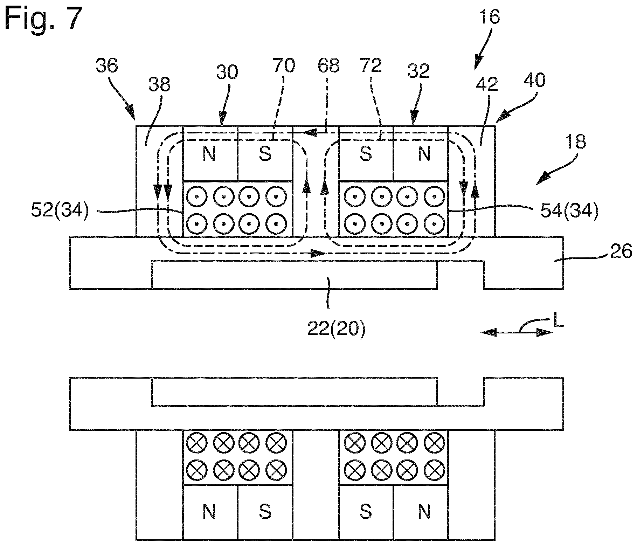

Der linke Läufer 22 und der rechte Läufer 24 umfassen jeweils wenigstens teilweise ein paramagnetisches und/oder ferromagnetisches Material. Mit anderen Worten sind also die Läufer 22, 24 zumindest teilweise aus einem paramagnetischen und/oder einem ferromagnetischen Material hergestellt. So ist es möglich, die Läufer 22, 24 durch Beaufschlagung mit einem elektromagnetischen Feld 68 in der jeweiligen Längsaxialrichtung LL, LR in dem zugehörigen Führungsrohr 26, 28 zu verschieben. Der Stator 18 umfasst einen distalen Permanentmagneten 30 und einen proximalen Permanentmagneten 32. Die beiden Permanentmagneten 30, 32 sind in Längsaxialrichtung L gegensinnig gepolt. Weitere Einzelheiten hierzu werden im Zusammenhang mit den Figuren 6 und 7 erläutert.

Ferner umfasst der Stator 18 eine elektrische Spule zum Erzeugen des elektronmagnetischen Felds 68. In Figur 2 ist diese Spule nur teilweise sichtbar. Das von ihr erzeugte elektromagnetische Feld 68 dient dazu, die Läufer 22, 24 in ihren Führungsrohren 26, 28 entlang der jeweiligen Längsaxialrichtung LL, LR zu verschieben.

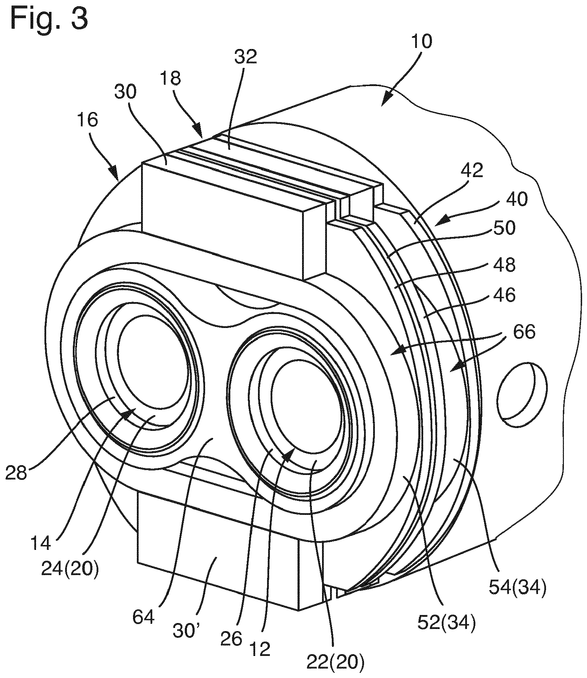

Figur 3 zeigt das aus Figur 2 bekannte stereoskopische optische System 10 ebenfalls in einer vereinfachten perspektivischen Ansicht und in einer Einbausituation an einem distalen Ende 6 eines Endoskopschafts 4. Um den Blick auf die dahinterliegenden Bauteile, insbesondere auf die eben erwähnte elektrische Spule 34, freizugeben, ist ein proximaler Statorpolschuh nicht dargestellt.

Der Stator 18 des in den Figuren 2 und 3 gezeigten stereoskopischen optischen Systems 10 umfasst an seinem distalen Ende 36 einen distalen Statorpolschuh 38. An seinem in Längsaxialrichtung L gegenüberliegenden proximalen Ende 40 umfasst der Stator 18 einen proximalen Statorpolschuh 42. Der Stator 18 umfasst außerdem einen zentralen Statorpolschuh 44, der in Längsaxialrichtung L zwischen den Permanentmagneten 30, 32 angeordnet ist und in dem gezeigten Ausführungsbeispiel aus einem proximalen zentralen Stator-Teil-Polschuh 46 und aus einem distalen zentralen Stator-Teil-Polschuh 48 gebildet ist. Zwischen dem proximalen zentralen Stator-Teil-Polschuh 46 und dem distalen zentralen Stator-Teil-Polschuh 48 ist beispielsweise ein Luftspalt 50 vorhanden. Die Spule 34 ist in eine distale Spule 52 und in eine proximale Spule 54 untereilt.

Der distale Statorpolschuh 38, die distale Spule 52, der distale Permanentmagnet 30 und der distale zentrale Stator-Teil-Polschuh 48 bilden eine vorgefertigte distale Baugruppe 60. Der proximale zentrale Stator-Teil-Polschuh 46, die proximale Spule 54, der proximale Permanentmagnet 32 und der proximale Statorpolschuh 42 bilden eine proximale Baugruppe 62. Die Bauteile der distalen Baugruppe 60 sind beispielsweise miteinander verklebt. Gleiches gilt für die Bauteile der proximalen Baugruppe 62. So ist es möglich, dass vorgefertigte Baugruppen 60, 62 bereitgestellt werden und aus diesen das stereoskopische optische System, genauer dessen Stator 18, zusammengesetzt wird. Es ist in diesem Zusammenhang beispielsweise vorgesehen, dass die beiden Baugruppen 60, 62 identisch vorgefertigt werden. Der Unterschied zwischen der distalen Baugruppe 60 und der proximalen Baugruppe 62 ist lediglich die Polung, d. h. die Ausrichtung, von deren Permanentmagneten 30, 32. Um eine entgegengesetzte Orientierung der Permanentmagnete 30, 32 der beiden Baugruppen 60, 62 bereitzustellen, kann eine der beiden Baugruppen 60, 62 um 180° gedreht zu der anderen Baugruppe 60, 62 eingebaut werden.

Die Permanentmagneten 30, 32, welche in die Baugruppen 60, 62 integriert sind, sind beispielsweise blockförmige Magneten. Diese sind ferner beispielsweise in mehreren Gruppen angeordnet, wobei die Gruppen bevorzugt an einander gegenüberliegenden Positionen angeordnet sind. Im dargestellten Ausführungsbeispiel sind die Permanentmagneten 30, 32 in zwei Gruppen angeordnet. So umfasst der distale Permanentmagnet den mit Bezugszeichen 30 und Bezugszeichen 30' bezeichneten Magnetblock. Der proximale Permanentmagnet 32 umfasst den oberhalb in Längsaxialrichtung L proximal hinter dem distalen Permanentmagneten 30 vorhandenen Block 32 sowie einen weiteren, in den Figuren nicht sichtbaren, Magnetblock 32', der sich in Längsaxialrichtung L proximal hinter dem distalen Permanentmagnetblock 30' befindet. Die Permanentmagneten 30, 32 sind in Längsaxialrichtung L gegensinnig gepolt. Dies bedeutet, dass sich die Magnetblöcke gegenseitig abstoßen. Diese abstoßende Kraft sorgt dafür, dass die distale Baugruppe 60 und die proximale Baugruppe 62 auseinandergedrückt werden, so dass zwischen ihnen der Luftspalt 50 verbleibt. Die distale Baugruppe 60 wird an einen distalen Anschlag gedrückt, während die proximale Baugruppe 62 an einen proximalen Anschlag gedrückt wird. Vorteilhaft erfolgt die Ausrichtung der Baugruppen 60, 62 ohne dass die erreichte Position von Bauteiltoleranzen abhängig ist.

Es ist nicht erforderlich, dass die beiden Baugruppen 60, 62 in der Einbausituation miteinander verbunden werden. Die zwischen den Baugruppen wirkenden abstoßenden Magnetkräfte sind groß genug, um die Baugruppen 60, 62 am Platz zu halten. Sollen die Baugruppen 60, 62 fixiert werden, so wird ein Klebstoff eingesetzt, der eine besonders geringe Volumenschrumpfung (beispielsweise weniger als 5 Vol.-%) während des Aushärtvorgangs zeigt.

Die Gruppen der blockförmigen Permanentmagneten sind jeweils an einer flachen Seite einer aus dem rechten und linken Führungsrohr 26, 28 gebildeten Anordnung angeordnet. Dies zeigt Figur 3 besonders deutlich.

Das linke Führungsrohr 26 und das rechte Führungsrohr 28 sind in einem gemeinsamen Bauteil 64 aufgenommen. Wie bereits erwähnt, kann es sich bei dem linken Führungstor 26 und/oder dem rechten Führungsrohr 28 um separate Bauteile, beispielsweise Rohre, handeln. Es ist jedoch ebenso vorgesehen, dass das linke Führungsrohr 26 und/oder das rechte Führungsrohr 28 als Bohrungen in das gemeinsame Bauteil 64 eingelassen sind. Dieses gemeinsame Bauteil 64 weist in einer Ebene quer zu der Längsaxialrichtung L einen hantelförmigen Querschnitt auf. Eine Innenkontur der Polschuhe 38, 48, 46, 42 entspricht einer Außenkontur des hantelförmigen Bauteils 64. Eine Außenkontur der Polschuhe 38, 48, 46, 42 ist zumindest abschnittsweise kreissegmentförmig. Wie Figur 2 und 3 zeigen, befinden sich diese Abschnitte der Polschuhe 38, 48, 46, 42 an den Stirnseiten der aus dem linken und rechten Führungsrohr 26, 28 gebildeten Anordnung und nicht an dessen Flachseiten.

Die Spule 34, d. h. die distale Spule 52 und die proximale Spule 54, umgeben das linke und rechte Führungsrohr 26, 28 und sind in einer quer zu der Längsaxialrichtung L gelegenen Ebene oval. Die Permanentmagneten 30, 32 sind auf einer den Führungsrohren 26, 28 abgewandten Außenseite 66 der Spule 34 angeordnet. Genauer gesagt ist der distale Permanentmagnet 30, 30' auf einer Außenseite 66 der distalen Spule 52 angeordnet und ein proximaler Permanentmagnet 32 ist auf der Außenseite 66 einer proximalen Spule 54 angeordnet.

Die Permanentmagneten 30, 32 bilden aufgrund ihrer Anordnung magnetische Rückschlusselemente für das von der elektrischen Spule 34 erzeugte elektromagnetische Feld 68.

Beispielsweise sind die Permanentmagneten 30, 30', 32, 32' oder auch nur einer dieser Permanentmagnete aus einer Kunststoffmatrix hergestellt, in die magnetische Partikel, insbesondere hartmagnetische Partikel, eingebettet sind. Ein solcher Permanentmagnet lässt sich insbesondere in einem Spritzgussverfahren herstellen. Vorteilhaft kann bei Herstellung des Permanentmagneten 30, 30', 32, 32' nicht nur der Permanentmagnet selbst hergestellt werden, sondern es können auch die Bauteile der entsprechenden Baugruppe 60, 62 miteinander verbunden werden. Es ist ferner vorteilhaft, wenn ein Spulendraht der Spule 34 durch den Permanentmagneten 30, 30', 32, 32' hindurchgeführt wird, also mit anderen Worten der Spulendraht mit eingegossen wird.

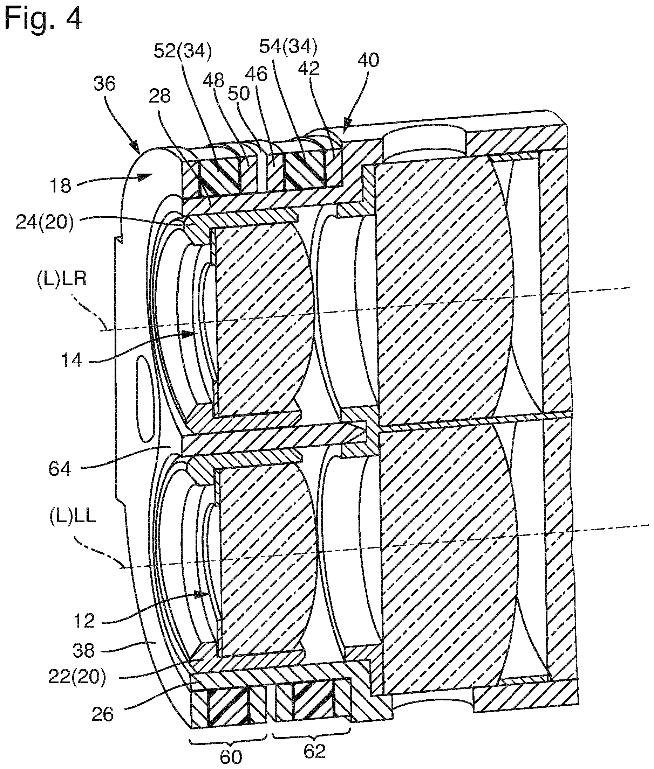

Figur 4 zeigt das stereoskopische optische System 10 in einer schematisch vereinfachten Schnittansicht in einer Ebene, in der eine Verbindungslinie zwischen den beiden optischen Kanälen 12, 14 liegt. In dieser Ebene liegen auch die Längsaxialrichtungen LL, LR des linken optischen Kanals 12 und des rechten optischen Kanals 14.

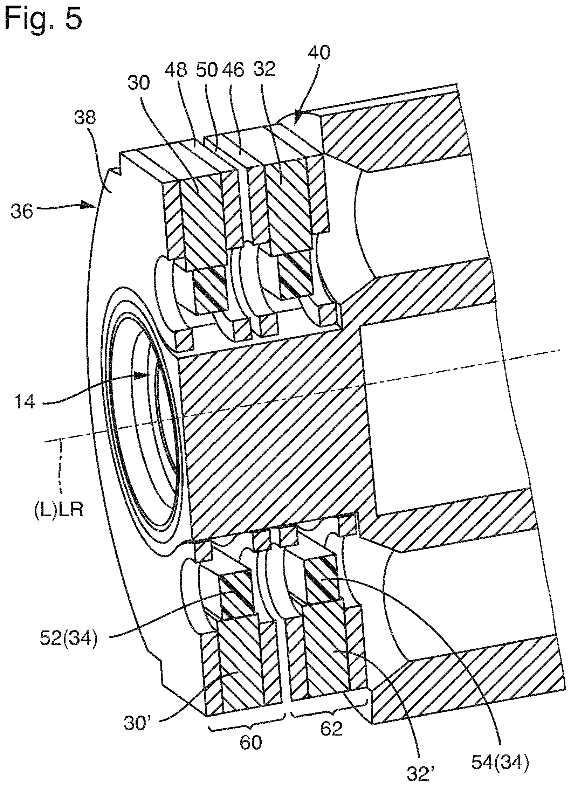

Figur 5 zeigt eine weitere schematische Schnittansicht in einer Ebene, welche senkrecht zu derjenigen Ebene liegt, in der die in Figur 4 dargestellte Schnittansicht liegt.

Die Funktionsweise des elektromagnetischen Aktuators des stereoskopischen optischen Systems 10 wird im Folgenden unter Bezugnahme auf die schematisch vereinfachten Prinzipskizzen der Figuren 6 und 7 erläutert.

Figur 6 zeigt den elektromagnetischen Aktuator 16 in unbestromtem Zustand, in dem sich dessen Läufer, bei dem es sich lediglich beispielhaft um den linken Läufer 22 handeln soll, in einer proximalen Endposition befindet. Der Läufer 22 ist innerhalb des linken Führungsrohres 26 in Längsaxialrichtung L verschiebbar aufgenommen. Außerhalb des Führungsrohres 26 befindet sich der Stator 18 des elektromagnetischen Aktuators 16. Dargestellt sind ferner der distale Permanentmagnet 30 und der proximale Permanentmagnet 32, welche gegensätzlich gepolt sind. Die Nord-Süd-Richtungen der Permanentmagnete 30, 32 liegen parallel zu der Längsaxialrichtung L. An einem distalen Ende 36 des Stators 18 befindet sich ein distaler Statorpolschuh 38, an einem proximalen Ende 40 des Stators 18 befindet sich ein proximaler Statorpolschuh 42. In Längsaxialrichtung L zwischen den Permanentmagneten 30, 32 befindet sich ein zentraler Statorpolschuh 44. Die Spule 34 umfasst eine distale Spule 52 und eine proximale Spule 54.

Figur 7 zeigt den elektromagnetischen Aktuator 16, wobei die distale Spule 52 und die proximale Spule 54 bestromt sind. Die beiden Spulen 52, 54 sind so miteinander gekoppelt, dass ein von der jeweiligen Spule 52, 54 erzeugtes erstes Magnetfeld und zweites Magnetfeld gleich orientiert sind. Dies ergibt sich aus der gleichen Bestromung der beiden Spulen 52, 54. Die Stromflussrichtung ist in den schematisch skizzierten Leitern der Spulen 52, 54 angedeutet. Eine aus der Zeichenebene herauszeigende Stromrichtung ist durch einen Punkt und eine in die Zeichenebene hineingerichtete Stromrichtung durch ein Kreuz angedeutet. Das erste und zweite Magnetfeld der Spulen 52, 54 addieren sich zu dem elektromagnetischen Feld 68, welches in strichpunktierter Linie dargestellt ist.

Das elektromagnetische Feld 68 überlagert ein erstes statisches Magnetfeld 70, welches von dem distalen Permanentmagneten 30 erzeugt wird und ein zweites statisches Magnetfeld 72, welches von dem zweiten Permanentmagneten 32 erzeugt wird. Am distalen Ende 36 des Stators 18 überlagern sich das elektromagnetische Feld 68 und das erste statische Magnetfeld 70 konstruktiv, so dass durch die Bestromung der Spule 34 an dieser Seite des Stators 18 eine Verstärkung des insgesamt vorliegenden Magnetfelds eintritt. Am proximalen Ende 40 des Stators 18 sind das elektromagnetische Feld 68 und das zweite statische Magnetfeld 72, welches von dem proximalen Permanentmagneten 32 erzeugt wird, entgegengerichtet, so dass an diesem Ende des Stators 18 eine Abschwächung des in Summe vorliegenden Magnetfelds stattfindet. So wirkt in einem Spalt (der in der dargestellten Situation geschlossen ist) zwischen dem Läufer 22 und dem Führungsrohr 26 an dem distalen Ende 36 eine größere Kraft als am proximalen Ende 40, so dass sich der Läufer 20 in die in Figur 7 gezeigte Endposition verlagert. Eine Verlagerung zurück in die Ausgangsposition erfolgt durch entsprechend entgegengesetzte Bestromung der Spule 34.

In den Figuren 6 und 7 sind die entsprechenden Bauteile lediglich in der oberen Hälfte der Zeichnung dargestellt. Die dargestellte Schnittansicht ist jedoch zu einer mittleren Längsachse rotationssymmetrisch, weshalb die entsprechenden Bauteile auch auf der unteren Hälfte der jeweiligen Zeichnung analog zu finden sind.

Die Erfindung ist in den folgenden Ansprüchen definiert.

### Bezugszeichenliste

- 2: Endoskop
- 4: Endoskopschaft
- 6: distales Ende
- 8: Handgriff
- 10: stereoskopisches optisches System
- 12: linker optischer Kanal
- 14: rechter optischer Kanal
- 16: elektromagnetischer Aktuator
- 18: Stator
- 20: Läufer
- 22: linker Läufer
- 24: rechter Läufer
- 26: linkes Führungsrohr
- 28: rechtes Führungstor
- 30, 30': distaler Permanentmagnet
- 32, 32': proximaler Permanentmagnet
- 34: Spule
- 36: distales Ende des Stators
- 38: distaler Statorpolschuh
- 40: proximales Ende des Stators
- 42: proximaler Statorpolschuh
- 44: zentraler Statorpolschuh
- 46: proximaler zentraler Stator-Teil-Polschuh
- 48: distaler zentraler Stator-Teil-Polschuh
- 50: Luftspalt
- 52: distale Spule
- 54: proximale Spule
- 60: distale Baugruppe
- 62: proximale Baugruppe
- 64: hantelförmiges Bauteil
- 66: Außenseite
- 68: elektromagnetisches Feld
- 70: erstes statisches Magnetfeld
- 72: zweites statisches Magnetfeld

- L: Längsaxialrichtung
- LL: linke Längsaxialrichtung
- LR: rechte Längsaxialrichtung

## Patentansprüche

1. Stereoskopisches optisches System (10) für ein chirurgisches Instrument, das System umfassend einen linken optischen Kanal (12), einen rechten optischen Kanal (14) und einen elektromagnetischen Aktuator (16) mit einem Stator (18) und einem Läufer (20), wobei optische Komponenten des linken optischen Kanals (12) in einem linken Führungsrohr (26) und optische Komponenten des rechten optischen Kanals (14) in einem separaten rechten Führungsrohr (28) angeordnet sind, wobei der Läufer einen linken Läufer (22), in dem zumindest eine optische Komponente des linken optischen Kanals (12) aufgenommen ist, und einen rechten Läufer (24), in dem zumindest eine optische Komponente des rechten optischen Kanals (14) aufgenommen ist, umfasst, und wobei der linke und der rechte Läufer (22, 24) in einer Längsaxialrichtung (LL, LR) des linken und rechten Führungsrohres (26, 28) in dem jeweiligen Führungsrohr (26, 28) verschiebbar gelagert sind, wobei die Läufer (22, 24) jeweils wenigstens teilweise ein paramagnetisches und/oder ferromagnetisches Material umfassen und durch Beaufschlagung mit einem elektromagnetischen Feld (68) in der Längsaxialrichtung (LL, LR) verschiebbar sind, und wobei der Stator (18) eine elektrische Spule (34) zum Erzeugen des elektromagnetischen Felds umfasst;
**gekennzeichnet dadurch, dass**
der Stator (18) einen distalen Permanentmagneten (30) und einen proximalen Permanentmagneten (32) umfasst, die in Längsaxialrichtung (L) gegensinning gepolt sind; und wobei der Stator außerhalb der Führungsrohre angeordnet ist.

2. Stereoskopisches optisches System (10) nach Anspruch 1, wobei ein distales Ende (36) des Stators (18) von einem distalen Statorpolschuh (38) und einem in Längsaxialrichtung (L) gegenüberliegendes proximales Ende (40) von einem proximalen Statorpolschuh (42) gebildet sind und der Stator (18) außerdem einen zentralen Statorpolschuh (44) umfasst, der in Längsaxialrichtung (L) zwischen den Permanentmagneten (30, 32) angeordnet ist, wobei der zentrale Statorpolschuh (44) aus einem proximalen zentralen Stator-Teil-Polschuh (46) und aus einem distalen zentralen Stator-Teil-Polschuh (48) gebildet ist.

3. Stereoskopisches optisches System (10) nach Anspruch 2, wobei die Spule (34) eine distale Spule (52) und eine proximale Spule (54) umfasst, wobei der distale Statorpolschuh (38), die distale Spule (52), der distale Permanentmagnet (30) und der distale zentrale Stator-Teil-Polschuh (48) eine vorgefertigte distale Baugruppe (60) bilden und der proximale zentrale Stator-Teil-Polschuh (46), die proximale Spule (54), der proximale Permanentmagnet (32) und der proximale Statorpolschuh (42) eine vorgefertigte proximale Baugruppe (62) bilden, wobei insbesondere die Bauteile der distalen und/oder der proximalen Baugruppe (60, 62) miteinander verklebt sind.

4. Stereoskopisches optisches System (10) nach Anspruch 2 oder 3, wobei das linke und das rechte Führungsrohr (26, 28) in einem gemeinsamen Bauteil (64) aufgenommen sind, welches in einer Ebene quer zu der Längsaxialrichtung (L) einen hantelförmigen Querschnitt aufweist und wobei eine Innenkontur der Polschuhe (38, 48, 46, 42) einer Außenkontur des hantelförmigen Bauteils (64) entspricht und eine Außenkontur der Polschuhe (38, 48, 46, 42) zumindest abschnittsweise kreissegmentförmig ist.

5. Stereoskopisches optisches System (10) nach einem der Ansprüche 1 bis 4, wobei die Spule (34) das linke und das rechte Führungsrohr (26, 28) umgibt und in einer quer zu der Längsaxialrichtung (L) gelegenen Ebene oval ist.

6. Stereoskopisches optisches System (10) nach einem der Ansprüche 1 bis 5, wobei die Permanentmagneten (30, 32) auf einer den Führungsrohren (26, 28) abgewandten Außenseite (66) der Spule (34) angeordnet sind.

7. Stereoskopisches optisches System (10) nach einem der Ansprüche 1 bis 6, wobei die Permanentmagneten (30, 32) blockförmige Magnete sind, die in zwei Gruppen angeordnet sind, wobei die Gruppen einander gegenüberliegend an je einer flachen Seite einer aus dem linken und rechten Führungsrohr (26, 28) gebildeten Anordnung angeordnet sind.

8. Stereoskopisches optisches System (10) nach einem der Ansprüche 1 bis 7, wobei die Permanentmagneten (30, 32) magnetische Rückschlusselemente für das von der elektrischen Spule (34) erzeugte Magnetfeld bilden.

9. Stereoskopisches optisches System (10) nach einem der Ansprüche 1 bis 8, wobei zumindest einer der Permanentmagnete (30, 30', 32, 32') hartmagnetische Partikel umfasst, die in einer Kunststoffmatrix eingebettet sind, wobei dieser Permanentmagnet (30, 30', 32, 32') insbesondere in einem Spritzgussverfahren hergestellt ist und ferner insbesondere in zumindest einem Permanentmagneten zumindest ein Spulendraht der Spule (34) eingegossen ist.

10. Chirurgisches Instrument, insbesondere Endoskop (2), mit einem stereoskopischen optischen System (10) nach einem der Ansprüche 1 bis 9.

11. Verfahren zum Herstellen eines stereoskopischen optischen Systems (10) für ein chirurgisches Instrument, mit einem linken optischen Kanal (12), einem rechten optischen Kanal (14) und einem elektromagnetischen Aktuator (16) mit einem Stator (18) und einem Läufer (20), **dadurch gekennzeichnet, dass** optische Komponenten des linken optischen Kanals (12) in einem linken Führungsrohr (26) und optische Komponenten des rechten optischen Kanals (14) in einem separaten rechten Führungsrohr (28) angeordnet werden, wobei der Stator (18) außerhalb der Führungsrohre (26, 28) angeordnet wird und der Läufer (20) einen linken Läufer (22), in dem zumindest eine optische Komponente des linken optischen Kanals (12) aufgenommen wird, und einen rechten Läufer (24), in dem zumindest eine optische Komponente des rechten optischen Kanals (14) aufgenommen wird, umfasst, und wobei der linke und der rechte Läufer (22, 24) in einer Längsaxialrichtung (L) des linken und rechten Führungsrohres (26, 28) in dem jeweiligen Führungsrohr (26, 28) verschiebbar gelagert werden, wobei die Läufer (22, 24) jeweils wenigstens teilweise ein paramagnetisches und/oder ferromagnetisches Material umfassen und durch Beaufschlagung mit einem elektromagnetischen Feld (68) in der Längsaxialrichtung (L) verschiebbar sind, wobei im Stator (18) ein distaler Permanentmagnet (30) und ein proximaler Permanentmagnet (32) derart angeordnet werden, dass sie in Längsaxialrichtung (L) gegensinnig gepolt sind, und wobei im Stator (18) eine elektrische Spule (34) zum Erzeugen des elektromagnetischen Felds (68) angeordnet wird.

12. Verfahren nach Anspruch 11, wobei ein distales Ende (36) des Stators (18) von einem distalen Statorpolschuh (38) und ein in Längsaxialrichtung (L) gegenüberliegendes proximales Ende (40) von einem proximalen Statorpolschuh (42) gebildet sind und der Stator (18) einen zentralen Statorpolschuh (44) umfasst, der in Längsaxialrichtung (L) zwischen den Permanentmagneten (30, 32) angeordnet wird und aus einem proximalen zentralen Stator-Teil-Polschuh (46) und aus einem distalen zentralen Stator-Teil-Polschuh (48) gebildet wird, wobei eine distale Baugruppe (60) vorgefertigt wird, indem der distale Statorpolschuh (38), eine distale Spule (52), der distale Permanentmagnet (30) und der distale zentrale Stator-Teil-Polschuh (48) miteinander verklebt werden und eine proximale Baugruppe (62) vorgefertigt wird, indem der proximale zentrale Stator-Teil-Polschuh (46), eine proximale Spule (54), der proximale Permanentmagnet (32) und der proximale Statorpolschuh (42) miteinander verklebt werden.

13. Verfahren nach Anspruch 11 oder 12, wobei die Permanentmagneten (30, 32) blockförmige Magneten sind, wobei die Magneten in zwei Gruppen angeordnet werden und die Gruppe einander gegenüberliegend an je einer flachen Seite einer aus dem linken und rechten Führungsrohr (26, 28) gebildeten Anordnung angeordnet werden.

14. Verfahren nach einem der Ansprüche 11 bis 13, wobei zumindest einer der Permanentmagnete (30, 32) hergestellt wird, indem hartmagnetische Partikel in eine Kunststoffmatrix eingebettet werden, wobei dieser Permanentmagnet insbesondere in einem Spritzgussverfahren hergestellt wird und ferner insbesondere in zumindest einem Permanentmagneten (30, 32) zumindest ein Spulendraht der Spule (34) eingegossen wird.

## Claims

1. A stereoscopic optical system (10) for a surgical instrument, the system comprising a left optical channel (12), a right optical channel (14) and an electromagnetic actuator (16) with a stator (18) and a rotor (20), wherein optical components of the left optical channel (12) are arranged in a left guide tube (26) and optical components of the right optical channel (14) are arranged in a separate right guide tube (28), wherein the rotor comprises a left rotor (22), in which at least one optical component of the left optical channel (12) is accommodated, and a right rotor (24), in which at least one optical component of the right optical channel (14) is accommodated, and wherein the left and the right rotor (22, 24) are mounted in the respective guide tube (26, 28) such that they can move in a longitudinal axial direction (LL, LR) of the left and right guide tube (26, 28), wherein the rotors (22, 24) each at least partially comprise a paramagnetic and/or ferromagnetic material and can be moved in the longitudinal axial direction (LL, LR) by application of an electromagnetic field (68), and wherein the stator (18) comprises an electric coil (34) for generating the electromagnet field; **characterized in that**
the stator (18) comprises a distal permanent magnet (30) and a proximal permanent magnet (32) which are oppositely polarized in the longitudinal axial direction (L); and wherein the stator is arranged outside the guide tubes.

2. The stereoscopic optical system (10) according to Claim 1, wherein a distal end (36) of the stator (18) is formed by a distal stator pole shoe (38) and an opposite proximal end (40) in the longitudinal axial direction (L) is formed by a proximal stator pole shoe (42), and the stator (18) in addition comprises a central stator pole shoe (44) which is arranged between the permanent magnets (30, 32) in the longitudinal axial direction (L), wherein the central stator pole shoe (44) is formed from a proximal central stator part pole shoe (46) and from a distal central stator part pole shoe (48).

3. The stereoscopic optical system (10) according to Claim 2, wherein the coil (34) comprises a distal coil (52) and a proximal coil (54), wherein the distal stator pole shoe (38), the distal coil (52), the distal permanent magnet (30) and the distal central stator part pole shoe (48) form a prefabricated distal assembly (60) and the proximal central stator part pole shoe (46), the proximal coil (54), the proximal permanent magnet (32) and the proximal stator pole shoe (42) form a prefabricated proximal assembly (62), wherein the components of the distal and/or the proximal assembly (60, 62) are in particular bonded to one another.

4. The stereoscopic optical system (10) according to Claim 2 or 3, wherein the left and the right guide tube (26, 28) are accommodated in a joint component (64) which has a dumbbell-shaped cross-section in a plane transversely to the longitudinal axial direction (L), and wherein an inner contour of the pole shoes (38, 48, 46, 42) corresponds to an outer contour of the dumbbell-shaped component (64) and an outer contour of the pole shoes (38, 48, 46, 42) is in the form of a circular segment at least in sections.

5. The stereoscopic optical system (10) according to any one of Claims 1 to 4, wherein the coil (34) surrounds the left and the right guide tube (26, 28) and is oval in a plane oriented perpendicular to the longitudinal axial direction (L).

6. The stereoscopic optical system (10) according to any one of Claims 1 to 5, wherein the permanent magnets (30, 32) are arranged on an outer side (66) of the coil (34) facing away from the guide tubes (26, 28).

7. The stereoscopic optical system (10) according to any one of Claims 1 to 6, wherein the permanent magnets (30, 32) are block-shaped magnets which are arranged in two groups, wherein the groups are arranged opposite one another on a flat side each of an arrangement formed from the left and right guide tube (26, 28).

8. The stereoscopic optical system (10) according to any one of Claims 1 to 7, wherein the permanent magnets (30, 32) form magnetic return elements for the magnetic field generated by the electric coil (34).

9. The stereoscopic optical system (10) according to any one of Claims 1 to 8, wherein at least one of the permanent magnets (30, 30', 32, 32') comprises magnetically hard particles which are embedded in a plastic matrix, wherein said permanent magnet (30, 30', 32, 32') is in particular produced using an injection molding method and at least one coil wire of the coil (34) is additionally in particular molded in at least one permanent magnet.

10. A surgical instrument, in particular an endoscope (2), having a stereoscopic optical system (10) according to any one of Claims 1 to 9.

11. A method for producing a stereoscopic optical system (10) for a surgical instrument, having a left optical channel (12), a right optical channel (14) and an electromagnetic actuator (16) with a stator (18) and a rotor (20), **characterized in that** optical components of the left optical channel (12) are arranged in a left guide tube (26) and optical components of the right optical channel (14) are arranged in a separate right guide tube (28), wherein the stator (18) is arranged outside the guide tubes (26, 28) and the rotor (20) comprises a left rotor (22), in which at least one optical component of the left optical channel (12) is accommodated, and a right rotor (24), in which at least one optical component of the right optical channel (14) is accommodated, and wherein the left and the right rotor (22, 24) are mounted in the respective guide tube (26, 28) such that they can move in a longitudinal axial direction (L) of the left and right guide tube (26, 28), wherein the rotors (22, 24) each at least partially comprise a paramagnetic and/or ferromagnetic material and can be moved in the longitudinal axial direction (L) by application of an electromagnetic field (68), wherein a distal permanent magnet (30) and a proximal permanent magnet (32) are arranged in the stator (18) in such a way that they are oppositely polarized in the longitudinal axial direction (L), and wherein an electric coil (34) for generating the electromagnet field (68) is arranged in the stator (18).

12. The method according to Claim 11, wherein a distal end (36) of the stator (18) is formed by a distal stator pole shoe (38) and an opposite proximal end (40) in the longitudinal axial direction (L) is formed by a proximal stator pole shoe (42), and the stator (18) comprises a central stator pole shoe (44) which is arranged between the permanent magnets (30, 32) in the longitudinal axial direction (L) and is formed from a proximal central stator part pole shoe (46) and from a distal central stator part pole shoe (48), wherein a distal assembly (60) is prefabricated in that the distal stator pole shoe (38), a distal coil (52), the distal permanent magnet (30) and the distal central stator part pole shoe (48) are bonded to one another and a proximal assembly (62) is prefabricated in that the proximal central stator part pole shoe (46), a proximal coil (54), the proximal permanent magnet (32) and the proximal stator pole shoe (42) are bonded to one another.

13. The method according to Claim 11 or 12, wherein the permanent magnets (30, 32) are block-shaped magnets, wherein the magnets are arranged in two groups and the groups are arranged opposite one another on a flat side each of an arrangement formed from the left and right guide tube (26, 28).

14. The method according to any one of Claims 11 to 13, wherein at least one of the permanent magnets (30, 32) is produced in that magnetically hard particles are embedded in a plastic matrix, wherein said permanent magnet is in particular produced using an injection molding method and at least one coil wire of the coil (34) is additionally in particular molded in at least one permanent magnet (30, 32).

## Revendications

1. Système optique stéréoscopique (10) pour un instrument chirurgical, le système comprenant un canal optique gauche (12), un canal optique droit (14) et un actionneur électromagnétique (16) ayant un stator (18) et un rotor (20), des composants optiques du canal optique gauche (12) étant disposés dans un tube de guidage gauche (26) et des composants optiques du canal optique droit (14) étant disposés dans un tube de guidage droit (23) séparé, le rotor comprenant un rotor gauche (22), dans lequel est logé au moins un composant optique du canal optique gauche (12), et un rotor droit (24), dans lequel est logé au moins un composant optique du canal optique droit (14), et les rotors gauche et droit (22, 24) sont montés de manière à pouvoir coulisser dans une direction axiale longitudinale (LL, LR) des tubes de guidage gauche et droit (26, 28) dans les tubes de guidage respectifs (26, 28), les rotors (22, 24) comprenant chacun, au moins en partie, un matériau paramagnétique et/ou ferromagnétique et apte à être déplacé dans la direction axiale longitudinale (LL, LR) par l'application d'un champ électromagnétique (68), le stator (18) comprenant une bobine électrique (34) pour générer le champ électromagnétique ;
**caractérisé en ce que**
le stator (18) comprend un aimant permanent distal (30) et un aimant permanent proximal (32) qui sont polarisés de manière opposée dans la direction axiale longitudinale (L) ; et le stator est disposé à l'extérieur des tubes de guidage.

2. Système optique stéréoscopique (10) selon la revendication 1, dans lequel une extrémité distale (36) du stator (18) est formée par une pièce polaire distale (38) de stator et une extrémité proximale (40) opposée dans la direction axiale longitudinale (L) est formée par une pièce polaire proximale (42) de stator, et le stator (18) comprend en outre une pièce polaire centrale (44) de stator, disposée entre les aimants permanents (30, 32) dans la direction axiale longitudinale (L), la pièce polaire centrale (44) de stator étant formée par une pièce polaire de partie centrale proximale (46) de stator et par une pièce polaire de partie centrale distale (48) de stator.

3. Système optique stéréoscopique (10) selon la revendication 2, dans lequel la bobine (34) comprend une bobine distale (52) et une bobine proximale (54), dans lequel la pièce polaire distale (38) de stator, la bobine distale (52), l'aimant permanent distal (30) et la pièce polaire de partie centrale distale (48) de stator forment un ensemble distal préfabriqué (60), et la pièce polaire de partie centrale proximale (46) de stator, la bobine proximale (54), l'aimant permanent proximal (32) et la pièce polaire proximale (42) de stator forment un ensemble proximal préfabriqué (62), les composants de l'ensemble distal et/ou proximal (60, 62) étant notamment liés ensemble.

4. Système optique stéréoscopique (10) selon la revendication 2 ou la revendication 3, dans lequel les tubes de guidage gauche et droit (26, 28) sont aménagés dans un composant commun (64) qui présente une section transversale en forme d'haltère dans un plan transversal à la direction de l'axe longitudinal (L), et dans lequel un contour intérieur des pièces polaires (38, 48, 46, 42) correspond à un contour extérieur du composant en forme d'haltère (64) et un contour extérieur des pièces polaires (38, 48, 46, 42) est en forme de segments de cercle au moins par portions.

5. Système optique stéréoscopique (10) selon l'une quelconque des revendications 1 à 4, dans lequel la bobine (34) entoure les tubes de guidage gauche et droit (26, 28) et est ovale dans un plan transversal à la direction axiale longitudinale (L).

6. Système optique stéréoscopique (10) selon l'une quelconque des revendications 1 à 5, dans lequel les aimants permanents (30, 32) sont disposés sur un côté extérieur (66) de la bobine (34) opposé aux tubes de guidage (26, 28).

7. Système optique stéréoscopique (10) selon l'une quelconque des revendications 1 à 6, dans lequel les aimants permanents (30, 32) sont des aimants en forme de bloc disposés en deux groupes, les groupes étant disposés en face l'un de l'autre sur un côté plat d'un ensemble formé par les tubes de guidage gauche et droit (26, 28).

8. Système optique stéréoscopique (10) selon l'une quelconque des revendications 1 à 7, dans lequel les aimants permanents (30, 32) forment des éléments de reflux magnétique pour le champ magnétique généré par la bobine électrique (34).

9. Système optique stéréoscopique (10) selon l'une quelconque des revendications 1 à 8, dans lequel au moins l'un des aimants permanents (30, 30', 32, 32') comprend des particules magnétiques dures enrobées dans une matrice en matière plastique, ledit aimant permanent (30, 30', 32, 32') étant en particulier fabriqué par un procédé de moulage par injection, et, en outre, en particulier, au moins un fil de bobine de la bobine (34) étant noyé dans au moins un aimant permanent.

10. Instrument chirurgical, en particulier endoscope (2), comportant un système optique stéréoscopique (10) selon l'une quelconque des revendications 1 à 9.

11. Procédé de fabrication d'un système optique stéréoscopique (10) pour un instrument chirurgical, comprenant un canal optique gauche (12), un canal optique droit (14) et un actionneur électromagnétique (16) comprenant un stator (18) et un rotor (20), **caractérisé en ce que** les composants optiques du canal optique gauche (12) sont disposés dans un tube de guidage gauche (26) et les composants optiques du canal optique droit (14) sont disposés dans un tube de guidage droit (28) séparé, le stator (18) étant disposé à l'extérieur des tubes de guidage (26, 28) et le rotor (20) comprenant un rotor gauche (22), dans lequel est logé au moins un composant optique du canal optique gauche (12), et un rotor droit (24), dans lequel est logé au moins un composant optique du canal optique droit (14), et les rotors gauche et droit (22, 24) sont supportés de manière à pouvoir coulisser dans une direction axiale longitudinale (L) des tubes de guidage gauche et droit (26, 28) dans les tubes de guidage respectifs (26, 28), les rotors (22, 24) comprenant chacun, au moins en partie, un matériau paramagnétique et/ou ferromagnétique et apte à être déplacé dans la direction de l'axe longitudinal (L) par l'application d'un champ électromagnétique (68), un aimant permanent distal (30) et un aimant permanent proximal (32) étant disposés dans le stator (18) de telle sorte qu'ils sont polarisés de manière opposée dans la direction de l'axe longitudinal (L), et une bobine électrique (34) étant disposée dans le stator (18) pour générer le champ électromagnétique (68).

12. Procédé selon la revendication 11, dans lequel une extrémité distale (36) du stator (18) est formée par une pièce polaire distale (38) de stator et une extrémité proximale (40) opposée dans la direction axiale longitudinale (L) est formée par une pièce polaire proximale (42) de stator, et le stator (18) comprend une pièce polaire centrale (44) de stator, disposée dans la direction axiale longitudinale (L) entre les aimants permanents (30, 32) et formée par une pièce polaire de partie centrale proximale (46) de stator et par une pièce polaire de partie centrale distale (48) de stator, un ensemble distal (60) étant préfabriqué en liant ensemble la pièce polaire distale (38) de stator, une bobine distale (52), l'aimant permanent distal (30) et la pièce polaire de partie centrale distale (48) de stator et un ensemble proximal (62) étant préfabriqué en liant ensemble la pièce polaire de partie centrale proximale (46) de stator, une bobine proximale (54), l'aimant permanent proximal (32) et la pièce polaire proximale (42) de stator.

13. Procédé selon la revendication 11 ou la revendication 12, dans lequel les aimants permanents (30, 32) sont des aimants en forme de bloc, les aimants étant disposés en deux groupes et chaque groupe est disposé à l'opposé l'un de l'autre sur chaque côté plat d'un ensemble formé par les tubes de guidage gauche et droit (26, 28).

14. Procédé selon l'une quelconque des revendications 11 à 13, dans lequel au moins l'un des aimants permanents (30, 32) est fabriqué par enrobage de particules magnétiques dures dans une matrice en matière plastique, cet aimant permanent étant fabriqué en particulier par un procédé de moulage par injection et, en outre, en particulier, au moins un fil de la bobine (34) étant noyé dans au moins un aimant permanent (30, 32).
